# EUROPEAN PATENT APPLICATION

(11) **EP 2 666 414 A1**
(43) Date of publication of application: **27.11.2013**
(21) Application number: 12075049.2
(22) Date of filing: 22.05.2012
(51) Int. Cl.: A61B 8/08, A61B 17/34

(54) **Ultrasound device for guided puncture of vascular access**

(71) Applicant: Zijlstra, Godert, 1215 GB Hilversum (NL); Zijlstra, Jan, 1251 XN Laren (NL)
(72) Inventor: Zijlstra, Godert, 1215 GB Hilversum (NL); Zijlstra, Jan, 1251 XN Laren (NL)

(57) **Abstract**

The invention relates to an ultrasound device, in particular an ultrasound device that is designed for vascular imaging, especially for guiding vascular puncture. It comprises an ultrasound imaging probe that can be stabilized at the point of puncture by integrated straps or by a handle designed to rest the probe like a stethoscope on the skin as to enable puncture at suitable spots of the human body to gain access to the vascular system. In this way the major problem for lesser trained hands to combine the plane of imaging of the probe with the imaging plane of the needle is overcome. The manual puncture process is aided by the ultrasound device by showing an image of the vessel to be punctured both in a longitudinal and an axial way, which together with the stabilized position of the device, enables the manual puncture to be as accurate as possible.

## Description

The invention relates to an ultrasound device, in particular an ultrasound device that is designed for vascular imaging, especially for guiding vascular puncture. It comprises an ultrasound imaging probe that can be stabilized at the point of puncture by integrated straps or by a handle designed to rest the probe like a stethoscope on the skin as to enable puncture at suitable spots of the human body to gain access to the vascular system. In this way the major problem for lesser trained hands to combine the plane of imaging of the probe with the imaging plane of the needle is overcome. The manual puncture process is aided by the ultrasound device by showing an image of the vessel to be punctured both in a longitudinal and an axial way, which together with the stabilized position of the device, enables the manual puncture to be as accurate as possible.

In the prior art relating to vessel puncture under ultrasound guidance, the existing methods are mainly suited for specialist procedures. It comprises a traditional ultrasound probe on which a device is mounted that can guide a puncture needle in a fixated manner under an adjustable degree with the human body. This procedure involves a manual control over the ultrasound probe, with left or right hand, while using the other hand to control the puncture process. This known method, like any other method including a manually controlled ultrasound probe of any sort, requires extensive experience and coordination during the puncture process. Also the ultrasound probe and device are not specifically designed for vascular access and hence will be a general utility ultrasound device which will require even more specialist knowledge as to programming and adjusting for optimal image for the purpose of vascular access.

The invention aims at removing the restrictions of existing vena puncture with ultrasound guidance. It does so by having a probe which is both particularly designed for vascular imaging in typical access areas, both in terms of imaging depth as width of imaging area, and is equipped with e.g. fixating straps, or any other mechanism for stabilizing the probe, that will eliminate the need for coordinating the probe with a free hand. Furthermore both the probe and the imaging screen have indication marks that enable the alignment between the probe and the vessel that requires the puncture.

The result of this combination is that both hands can be used for the needle puncture process. Less imaging adjustment is required to get accurate ultrasound guidance and the alignment between vessel and needle tip at point of access of the human body is indicated, all leading to more accurate punctures that require less attempts and harm/discomfort for the patient.

The before mentioned ultrasound probe is new and innovative in the sense that the probe is designed in such a way that is can be stabilized in any of the regular vascular access points like e.g. armpits or wrists. This is achieved by integrating the contact point of the probe in a flat surface which allows for stable contact with the human body. Existing ultrasound probes, even if equipped with fixating straps, would not be able to give a stable and frozen image of the designated area. A key technological step forward is to enable the probe to have stable contact with the skin by fixating the probe, and not as in the prior art by fixating the needle guidance. A fixated probe with a stable image will enable a more controlled access to the vascular system and will require less professional knowledge and training by the medical practitioner, since they can use both hands, like in a non-guided vena puncture, to make the needle puncture.

To further aid the guidance and control during the vena puncture, the probe will have a clear puncture area shaped as a half circle retraction from the diameter of the probe. The flat surface of the extended probe that has contact with the puncture area has in that sense a tiny half circle gap. In the deepest part of the half circle, there is a small indicator that corresponds with an indicator line on the display. This way the probe can be aligned with the ultrasound image on screen to the extent that the indicator on the probe can be placed on top of the vein to be punctured by looking at the image on screen to see when the vein and the on screen indicator are aligned. If they are aligned on the image, the probe should be stabilized for example by fixating straps or by a dedicated handle to keep the alignment. At that point the indicator on the probe, being placed and fixed in position to the vein, will provide a clear puncture entry point for the needle.

After the needle has entered the body, the screen will aid the puncture by showing a double image. It shows the vein to be punctured in a longitudinal and axial way, to be able to keep alignment and direction with the indicators on screen and probe, and it will show a cross section view of the vessel to be able to assess entry of the vein with the needle and hence also when to stop the puncture from extending deeper into the body. The result is a full three dimensional assistance of the vena puncture by the indicators and the double image on screen.

The combined results of a ultrasound probe designed for stable contact with the puncture area, fixated in place so no further manual control on the probe is required, the indicator alignment in the needle entry area of the probe with the indicator on the screen, and the double image providing also a depth dimension to the ultrasound imaging, gives maximum control, i.e. free to use both hands to guide the needle, and maximum accuracy, i.e. visibility of the vein to be accessed, for gaining intravenous access.

In conclusion vesselpuncture for gaining access to the circulation of a patient is probably the most executed procedure in outpatient and clinical healthcare. Emergency departments and ambulances, hematology labs, operating theatres, Intensive care units, hemodialysis departments, oncology departments and many other places like CT scans and MR suites require easy an sure and fast single puncture of a vessel to guarantee access to the circulation. Ultrasound assistance making easy such punctures for even unpracticed staff will be a hughe step forward in daily medicine!!

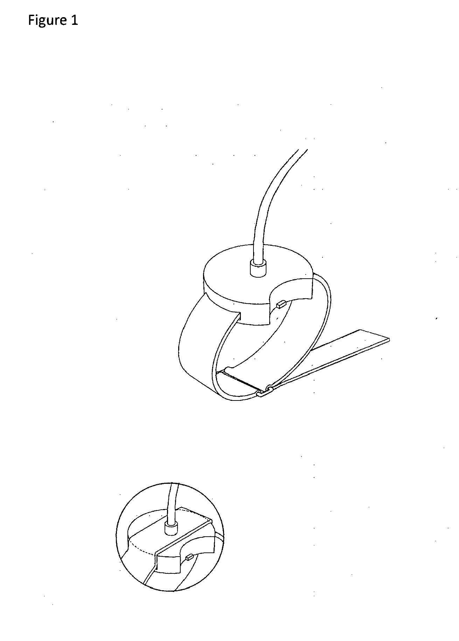

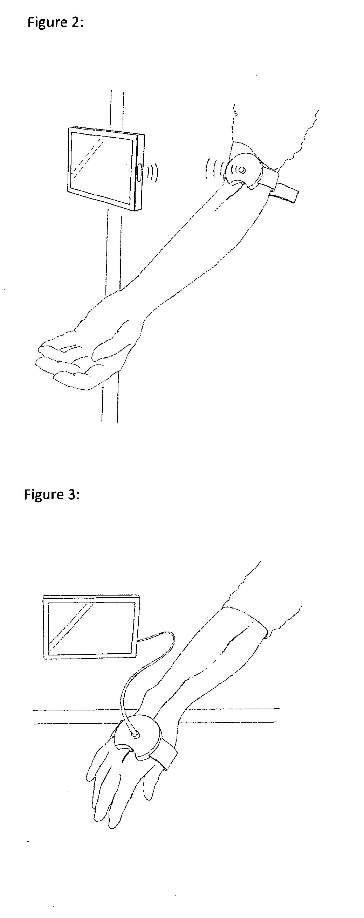

## Claims

1. A dedicated linear ultrasound probe for image guided vessel puncture. The probe comprises a flat surface contact area for application on the skin at the required puncture position to enable a stable position and image of the puncture area. The probe comprises of a retraction area in the surface dedicated for the precise puncture position without having to fix the needle to the probe, hence keeping all freedom of movement for guiding the needle into the vessel.

2. The imaging probe according to claim 1, **characterized by** the ability to fixate and stabilize the probe at the required skin level puncture area without manual interference. The fixating straps or a design with a fixed probe holder, or any other means of keeping the probe in position without using a hand, enables bimanual guidance of the needle puncture which relates best to the experience of personnel typically involved in vessel punctures.

3. The imaging probe according to claim 1, where the retraction area of the probe has an indicator at the deepest point of the retraction area that is aligned with an indicator at the image display to facilitate easy alignment of the longitudinal image of the vessel and the probe. After the indicator line on the screen is visually aligned with the image of the vessel by moving the probe over the skin, and the probe is fixed into position, the indicator on the probe provides an easy entry indicator for the needle puncture of the skin.

4. The imaging probe according to claim 1 provides longitudinal and axial simultaneous or alternative switchable imaging planes on the screen for maximum guidance of the needle entry into the vessel. After having aligned the indicators on the probe and the screen as in claim 3, and the needle has punctured the skin, the practitioner will have both hands free to control the needle to the point that it punctures the anterior vesselwall, and to the point that, after entering the vessel, the puncture stops to prevent the needle of going too deep and penetrating the posterior vesselwall with a consequent haematoma.

5. The imaging probe according to claim 1, with the addition of an easily exchangeable and sterile cover to enable the required level of patient hygiene, without having to sterilize the entire probe. This will enable a high usage rate of the imaging device, which is in line with the high number of vessel punctures performed in modern day healthcare practice.
